# EUROPÄISCHE PATENTSCHRIFT

(11) **EP 0 611 745 B1**
(45) Veröffentlichungstag und Bekanntmachung des Hinweises auf die Patenterteilung: **09.10.1996**
(21) Anmeldenummer: 94101812.9
(22) Anmeldetag: 07.02.1994
(51) Int. Cl.: C07C 17/25, C07C 17/00, C07C 21/18

(54) **Verfahren zur Herstellung von Hexafluorbuten**
Process for the preparation of hexafluorobutene
Procédé pour la préparation de l'hexafluorobutène

(30) Priorität: 19.02.1993 DE 4305163
(43) Veröffentlichungstag der Anmeldung: 24.08.1994
(73) Patentinhaber: BAYER AG, 51368 Leverkusen (DE)
(72) Erfinder: Lui, Norbert, Dr., D-51061 Köln (DE); Marhold, Albrecht, Dr., D-51373 Leverkusen (DE); Bielefeldt, Dietmar, Dr., D-40883 Ratingen (DE)

(56) Entgegenhaltungen:
- GB-A- 965 069
- US-A- 3 739 036
- US-A- 5 180 860

## Beschreibung

Die vorliegende Erfindung betrifft ein Verfahren zur Herstellung von 1,1,1,4,4,4-Hexafluorbuten-2 (CF₃-CH=CH-CF₃). 1,1,1,4,4,4-Hexafluorbuten-2 kann durch eine Hydrierung in 1,1,1,4,4,4-Hexafluorbutan überführt werden, das neuerdings Interesse als Treibmittel bei der Herstellung von Schaumstoffen hat, weil es dabei die ökologisch bedenklichen Fluor-Chlor-Kohlenwasserstoffe ersetzen kann.

Es ist bekannt, daß man durch Einwirkung von Kaliumfluorid auf Hexachlorbutadien in einem Carbonsäureamid-Lösungsmittel 1,1,1,2,4,4,4-Heptafluorbuten-2 erhalten kann (siehe US-A 3 287 425). Es war deshalb zu erwarten, daß sich bei der Einwirkung von Kaliumfluorid auf 1,1,2,4,4-Pentachlorbutadien unter den gleichen Bedingungen 1,1,1,4,4,4-Hexafluorbuten-2 herstellen lassen sollte. Es hat sich jedoch gezeigt, daß man so 1,1,1,4,4,4-Hexafluorbuten-2 nicht herstellen kann, denn es bilden sich bei dieser Reaktion nur harzartige Produkte (siehe Vergleichsbeispiel 1).

Außerdem wurde festgestellt, daß bei der Einwirkung von Kaliumfluorid auf 1,1,1-Trifluor-3,4,4-trichlorbuten-3 unter den oben genannten Bedingungen ebenfalls nur harzartige Produkte und nicht gemäß der US-A 3 287 425 zu erwartendes 1,1,1,4,4,4-Hexafluorbutan erhalten wird (siehe Vergleichsbeispiel 2).

Gemäß einem eigenen älteren, jedoch nicht vorveröffentlichten Vorschlag wird 1,1,2,4,4-Pentachlorbutadien mit Fluorwasserstoff in Gegenwart von Katalysatoren zunächst in 1,1,1,4,4,4-Hexafluor-2-chlorbutan umgesetzt und dieses durch Eliminierung und Hydrierung in 1,1,1,4,4,4-Hexafluorbutan überführt.

Etwas vereinfacht ergibt sich anhand von Reaktionsgleichungen dargestellt folgendes Bild: 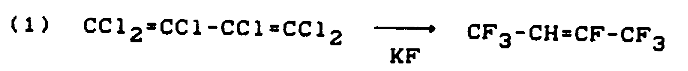 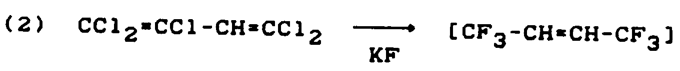 (zu erwarten aufgrund der US-A 3 287 425; tatsächlich: Harzbildung) 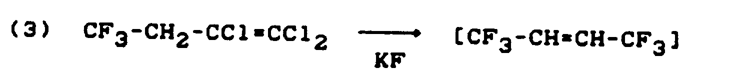 (zu erwarten aufgrund der US-A 3 287 425; tatsächlich: Harzbildung) 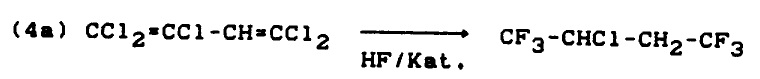 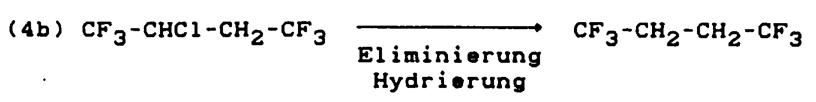 (eigener älterer, nicht vorveröffentlichter Vorschlag)

Die Herstellbarkeit von 1,1,1,4,4,4-Hexafluorbuten-2 und damit von 1,1,1,4,4,4-Hexafluorbutan durch Umsetzung einer chlorhaltigen C₄-Verbindung mit Kaliumfluorid ist demnach als wenig wahrscheinlich anzusehen.

Es wurde nun ein Verfahren zur Herstellung von 1,1,1,4,4,4-Hexafluorbuten-2 gefunden, das dadurch gekennzeichnet ist, daß man Chlor-Fluor-Butane der Formel (I)

CF₃-CH₂-CHCl-CX₃ (I),

in der die einzelnen Reste X unabhängig voneinander für Chlor und/oder Fluor stehen,
mit Alkalifluorid in einem aprotischen, polaren Lösungsmittel umsetzt.

Das erfindungsgemäße Verfahren läßt sich durch folgende Reaktionsgleichung beispielhaft illustrieren:

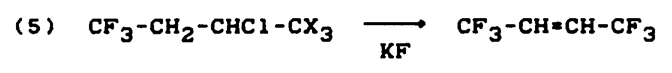

Für das erfindungsgemäße Verfahren geeignete Ausgangsprodukte der Formel (I) können beispielsweise erhalten werden, indem man 1,1,3,4,4-Pentachlorbutadien-1,3 mit Fluorwasserstoff in Gegenwart von Katalysatoren (z.B. Lewis-Säuren) umsetzt. Man erhält dabei im allgemeinen Gemische von Verbindungen der Formel (I), bei deren Komponenten die CX₃-Gruppe eine CCl₃-, CCl₂F-, CClF₂- oder eine CF₃-Gruppe sein kann. Es ist ein besonderer Vorteil des erfindungsgemäßen Verfahrens, daß man solche Gemische von Verbindungen der Formel (I) einsetzen kann, Natürlich kann man auch jeweils einzelne Verbindungen der Formel (I) einsetzen, beispielweise
1,1,1-Trifluor-3,4,4,4-tetrachlorbutan,
1,1,1,4-Tetrafluor-3,4,4-trichlorbutan,
1,1,1,4,4-Pentafluor-3,4-dichlorbutan oder
1,1,1,4,4,4-Hexafluor-3-chlorbutan.

Als Alkalifluorid kommen insbesondere Natrium- und Kaliumfluorid infrage, sowie deren Gemische mit wenig Cäsiumfluorid. Vorzugsweise wird Kaliumfluorid eingesetzt. Das Alkalifluorid kommt vorzugsweise in getrockneter Form zum Einsatz. Die Trocknung kann man beispielsweise durchführen, indem man es auf ca. 200 bis 490°C erhitzt oder, indem man es mit einem hochsiedenden Lösungsmittel versetzt, z.B. dem für die erfindungsgemäße Reaktion benötigten Lösungsmittel, und dann eine kleine Menge des Lösungsmittels zusammen mit eventuell vorhandenem Wasser abdestilliert.

Das Alkalifluorid kann beispielsweise in der stöchiometrisch erforderlichen Menge oder im Überschuß eingesetzt werden. Beim Einsatz von einem Mol reinem 1,1,1,4,4,4-Hexafluor-3-chlorbutan ist stöchiometrisch 1 Mol Alkalifluorid erforderlich, beim Einsatz von niedriger fluoriertem Ausgangsmaterial (in reiner Form oder im Gemisch mit 1,1,1,4,4,4-Hexafluor-3-chlorbutan) ist für jedes Mol weiterem, im Ausgangsmaterial vorhandenem Chlor stöchiometrisch ein weiteres Mol Alkalifluorid erforderlich bis hin zu 5 Molen Alkalifluorid beim Einsatz von reinem 1,1,1-Trifluor-3,4,4,4-tetrachlorbutan.

Vorzugsweise setzt man Alkalifluorid in einer Menge ein, die zwischen der stöchiometrisch erforderlichen Menge und einem 5-fachen molaren Überschuß davon liegt.

Als aprotische, polare Lösungsmittel kommen beispielsweise in Frage: Carbonsäureamide mit 1 bis 4 C-Atomen, N-C₁-C₄-Mono- und N-C₁-C₄-Dialkylderivate davon, C₁-C₄-Alkylsulfoxide, C₁-C₄-Sulfone, cyclische Alkylensulfone mit 5 bis 6 Ringatomen, cyclische Alkylencarbonate mit 5 bis 6 Ringatomen, und Lactone und Lactame mit jeweils 5 bis 7 Ringatomen. Bevorzugt sind Tetramethylensulfon, N-Methylpyrrolidon (NMP) und Dimethylacetamid.

Das aprotische, polare Lösungsmittel kann beispielsweise in Mengen von 25 bis 250 ml, bezogen auf 100 g eingesetzte Reaktanden (Alkalifluorid und Verbindungen der Formel (I)) verwendet werden.

Das erfindungsgemäße Verfahren kann beispielsweise bei Temperaturen im Bereich 120 bis 280°C durchgeführt werden. Bevorzugt sind Reaktionstemperaturen im Bereich 180 bis 230°C.

Das erfindungsgemäße Verfahren kann bei vermindertem, normalem oder erhöhtem Druck durchgeführt werden, wobei der Druck so gewählt werden kann, daß die Reaktionsprodukte in flüssiger Phase verbleiben oder während der Reaktion aus dem Reaktionsgemisch abdestillieren. Vorzugsweise arbeitet man bei Drucken zwischen Normaldruck und 100 bar.

Gegebenenfalls kann man das erfindungsgemäße Verfahren in Gegenwart von Phasentransferkatalysatoren durchführen. Hierfür kommen beispielsweise Kronenether und quartäre Stickstoff- oder Phosphorverbindungen in Frage. Phasentransferkatalysatoren kann man beispielsweise in Mengen von 0 bis 10 Gew.-%, bezogen auf eingesetztes Hexafluorbuten, einsetzen.

Während der Reaktion oder nach Beendigung der Reaktion kann man das hergestellte 1,1,1,4,4,4-Hexafluorbuten-2 durch Destillation oder auf andere Weise aus dem Reaktionsgemisch abtrennen.

Die Hydrierung von 1,1,1,4,4,4-Hexafluorbuten-2 zu 1,1,1,4,4,4-Hexafluorbutan kann beispielsweise auf an sich übliche katalytische Weise in flüssiger oder gasförmiger Phase erfolgen. Als Katalysatoren können übliche Hydrierkatalysatoren verwendet werden, beispielsweise solche, die Palladium, Nickel oder Verbindungen davon enthalten.

Im Hinblick auf den eingangs geschilderten Stand der Technik ist es ausgesprocxhen überraschend, daß es erfindungsgemäß gelingt, 1,1,1,4,4,4-Hexafluorbuten-2 auf einfache Weise und in guten Ausbeuten herzustellen.

### Beispiele

### Vergleichsbeispiel 1

Zu 1200 ml Tetramethylensulfon und 400 g getrocknetem Kaliumfluorid wurden bei 190°C 226 g 1,1,2,4,4-Pentachlorbutadien in 25 Minuten zugetropft. Nach 1 Stunde bei 220°C färbte sich das Reaktionsgemisch schwarz und verharzte. Es konnte kein 1,1,1,4,4,4-Hexafluorbuten-2 isoliert werden.

### Vergleichsbeispiel 2

Zu 350 ml Tetramethylensulfon und 80 g getrocknetem Kaliumfluorid wurden bei 190°C 50 g 1,1,1-Trifluor-3,4,4-trichlorbuten-3 zugetropft. Nach 1,5 Stunden bei 200 bis 220°C färbte sich das Reaktionsgemisch schwarz und verharzte. Es konnte kein 1,1,1,4,4,4-Hexafluorbuten-2 isoliert werden.

### Erfindungsgemäßes Beispiel 1

Zu einer Mischung von 3 l destillierten Tetramethylensulfon und 830 g getrocknetem Kaliumfluorid wurden bei 190°C 960 g 1,2-Dichlor-1,1,4,4,4-Pentafluorbutan zugetropft und 1,1,1,4,4,4-Hexafluorbuten-2 in dem Maße wie es sich bildete, abdestilliert. Das so erhaltene Produkt wurde redestilliert und so 650 g 1,1,1,4,4,4-Hexafluorbuten-2 mit einem Siedepunkt von 8°C bei Normaldruck erhalten.

### Erfindungsgemäßes Beispiel 2

Zu einer Mischung von 195 ml destilliertem Tetramethylensulfon und 87 g getrocknetem Kaliumfluorid wurden bei 190°C 200 g 2-Chlor-1,1,1,4,4,4-Hexafluorbutan zugetropft und das entstehende 1,1,1,4,4,4-Hexafluorbuten-2 kontinuierlich abdestilliert. Es wurden 135 g 1,1,1,4,4,4-Hexafluorbuten-2 erhalten.

## Patentansprüche

1. Verfahren zur Herstellung von 1,1,1,4,4,4-Hexafluorbuten-2, dadurch gekennzeichnet, daß man Chlor-Fluor-Butane der Formel (I)
CF₃-CH₂-CHCl-CX₃ (I),
in der die einzelnen Reste X unabhängig voneinander für Chlor und/oder Fluor stehen,
mit Alkalifluorid in einem aprotischen, polaren Lösungsmittel umsetzt.

2. Verfahren nach Anspruch 1, dadurch gekennzeichnet, daß man ein Gemisch von Verbindungen der Formel (I) einsetzt, bei deren Komponenten die CX₃-Gruppe eine CCl₃-, CCl₂F-, CClF₂- oder eine CF₃-Gruppe ist.

3. Verfahren nach Ansprüchen 1 bis 2, dadurch gekennzeichnet, daß man das Alkalifluorid in einer Menge zwischen der stöchiometrisch erforderlichen Menge und einem 5-fachen molaren Überschuß davon einsetzt.

4. Verfahren nach Ansprüchen 1 bis 3, dadurch gekennzeichnet, daß man als aprotisches, polares Lösungsmittel Carbonsäureamide mit 1 bis 4 C-Atomen, N-C₁-C₄-Mono- und N-C₁-C₄-Dialkylderivate davon, C₁-C₄-Alkylsulfoxide, C₁-C₄-Sulfone, cyclische Alkylensulfone mit 5 bis 6 Ringatomen, cyclische Alkylencarbonate mit 5 bis 6 Ringatomen oder Lactone oder Lactame mit jeweils 5 bis 7 Ringatomen einsetzt.

5. Verfahren nach Ansprüchen 1 bis 4, dadurch gekennzeichnet, daß man das aprotische, polare Lösungsmittel in Mengen von 25 bis 250 ml, bezogen auf 100 g eingesetzte Reaktanden, einsetzt.

6. Verfahren nach Ansprüchen 1 bis 5, dadurch gekennzeichnet, daß man es bei Temperaturen im Bereich von 120 bis 280°C durchführt.

7. Verfahren nach Ansprüchen 1 bis 6, dadurch gekennzeichnet, daß man es in Gegenwart von Phasentransferkatalysatoren durchführt.

8. Verfahren nach Ansprüchen 1 bis 7, dadurch gekennzeichnet, daß man während der Reaktion das hergestellte 1,1,1,4,4,4-Hexafluorbuten-2 durch Destillation abtrennt.

9. Verfahren nach Ansprüchen 1 bis 7, dadurch gekennzeichnet, daß man nach Beendigung der Reaktion das hergestellte 1,1,1,4,4,4-Hexafluorbuten-2 durch Destillation abtrennt.

## Claims

1. Process for preparing 1,1,1,4,4,4-hexafluorobut-2-ene, characterized in that chlorofluorobutanes of the formula (I)
CF₃-CH₂-CHCl-CX₃ (I)
in which the individual radicals X independently of each other represent chlorine and/or fluorine,
are reacted with alkali metal fluoride in an aprotic polar solvent.

2. Process according to Claim 1, characterized in that the compounds of the formula (I) are used in the form of a mixture in the components of which the CX₃ group is a CCl₃, CCl₂F, CClF₂ or a CF₃ group.

3. Process according to Claims 1 and 2, characterized in that the alkali metal fluoride is used in an amount between the stoichiometrically required amount and a 5-fold molar excess thereof.

4. Process according to Claims 1 to 3, characterized in that the aprotic polar solvents used are carboxamides having from 1 to 4 carbon atoms, N-C₁-C₄-mono- and N-C₁-C₄-dialkyl derivatives thereof, C₁-C₄-alkyl sulphoxides, C₁-C₄-sulphones, cyclic alkylene sulphones having from 5 to 6 ring atoms, cyclic alkylene carbonates having from 5 to 6 ring atoms, or lactones or lactams each having from 5 to 7 ring atoms.

5. Process according to Claims 1 to 4, characterized in that the aprotic polar solvent is used in amounts of from 25 to 250 ml, based on 100 g of reactants used.

6. Process according to Claims 1 to 5, characterized in that it is carried out at temperatures in the range from 120 to 280°C.

7. Process according to Claims 1 to 6, characterized in that it is carried out in the presence of phase transfer catalysts.

8. Process according to Claims 1 to 7, characterized in that during the reaction the 1,1,1,4,4,4-hexafluorobut-2-ene produced is separated off by distillation.

9. Process according to Claims 1 to 7, characterized in that after the end of the reaction the 1,1,1,4,4,4-hexafluorobut-2-ene produced is separated off by distillation.

## Revendications

1. Procédé de préparation du 1,1,1,4,4,4-hexafluorobutène-2, caractérisé en ce que l'on fait réagir des chloro-fluoro-butanes de formule (I) :
CF₃-CH₂-CHCl-CX₃ (I),
dans laquelle les divers symboles X représentent chacun, indépendamment les uns des autres, le chlore et/ou le fluor,
avec un fluorure alcalin dans un solvant polaire aprotonique.

2. Procédé selon la revendication 1, caractérisé en ce que l'on met en oeuvre un mélange de composés de formule (I) dont les composants contiennent en tant que groupe CX₃ un groupe CC1₃, CCl₂F, CClF₂ ou CF₃.

3. Procédé selon les revendications 1 et 2, caractérisé en ce que l'on met en oeuvre le fluorure alcalin en quantité comprise entre la quantité théorique et un excès de 5 mol/mol.

4. Procédé selon les revendications 1 à 3, caractérisé en ce que l'on utilise en tant que solvants polaires aprotoniques des carboxamides en C₁-C₄, leurs dérivés mono- et dialkylés en C₁-C₄ à l'azote, des alkylsulfoxydes en C₁-C₄, des sulfones en C₁-C₄, des alkylènesulfones cycliques contenant 5 à 6 atomes cycliques, des carbonates d'alkylènes cycliques contenant 5 à 6 atomes cycliques ou des lactones ou lactames contenant chacun 5 à 7 atomes cycliques.

5. Procédé selon les revendications 1 à 4, caractérisé en ce que le solvant polaire aprotonique est mis en oeuvre en quantité de 25 à 250 ml pour 100 g des réactifs mis en oeuvre.

6. Procédé selon les revendications 1 à 5, caractérisé en ce que l'on opère à des températures dans l'intervalle de 120 à 280°C.

7. Procédé selon les revendications 1 à 6, caractérisé en ce que l'on opère en présence de catalyseurs à transfert de phase.

8. Procédé selon les revendications 1 à 7, caractérisé en ce que l'on sépare le 1,1,1,4,4,4-hexafluorobutène-2 par distillation en cours de réaction.

9. Procédé selon les revendications 1 à 7, caractérisé en ce que l'on sépare le 1,1,1,4,4,4-hexafluorobutène-2 formé par distillation après la réaction.
